# FASCICULE DE BREVET EUROPEEN

(11) **EP 2 874 573 B1**
(45) Date de publication et mention de la délivrance du brevet: **02.09.2020**
(21) Numéro de dépôt: 13747437.5
(22) Date de dépôt: 09.07.2013
(51) Int. Cl.: A61F 2/46, A61B 17/32, A61B 17/16

(54) **ENSEMBLE D'ENTRAÎNEMENT POUR OUTIL DE COUPE DE TYPE SHAVER**
ANTRIEBSANORDNUNG FÜR EINE SCHNEIDEEINHEIT EINES RASIERAPPARATS
DRIVE ASSEMBLY FOR A SHAVER CUTTING UNIT

(30) Priorité: 19.07.2012 FR 1256963
(43) Date de publication de la demande: 27.05.2015
(73) Titulaire: Clariance, 62000 Dainville (FR)
(72) Inventeur: TORNIER, Alain, F-38330 Saint Ismier (FR); VIART, Guy, F-62128 Saint Leger (FR); LEROY, Jean Yves, F-62870 Campagne-Les-Hesdin (FR); KRIER, Brice, F-62000 Dainville (FR); BILLON, Adrien, F-59790 Rochin (FR); GANGI, Afshin, 77933 Lahr (DE); SCHULLER, Sébastien, F-67200 Strasbourg (FR); ORTIZ, Orlando, New York, New York 11530 (US)
(74) Mandataire: Godard, Xavier
(86) Numéro de dépôt international: PCT/FR2013/051631
(87) Numéro de publication internationale: WO 2014/013162

(56) Documents cités:
- WO-A1-2004/043271
- US-A1- 2004 010 258
- US-A1- 2010 152 758
- US-A1- 2010 152 762
- US-A1- 2010 217 245
- US-A1- 2010 234 866

## Description

La présente invention est relative à un appareil de nucléotomie permettant, à partir d'une voie d'abord trans-osseuse préalablement percée dans le corps d'une vertèbre, la réalisation dans un disque intervertébral d'un espace nucléique.

On connait d'après le brevet français N°11/00199 appartenant au demandeur un foret à profil courbe permettant la réalisation de la voie d'abord trans osseuse à l'intérieur du corps d'une vertèbre.

On connaît également d'après le brevet français N°08/01860 appartenant au demandeur un implant nucléique introduit à l'intérieur d'un espace nucléique ménagé dans le disque intervertébral.

Le document US2010/0217245 décrit un instrument médical comportant des moyens de transmission du mouvement rotatif issu du moteur à un outil et l'agencement d' aimants qui permettent uniquement l'entraînement en rotation de l'outil et dans un sens contraire à celui du moteur.

L'appareil de nucléotomie suivant la présente invention a pour objet de permettre au chirurgien la réalisation, après le perçage d'un ou deux canaux à l'intérieur du corps de la vertèbre, d'un espace nucléique pour la mise en place d'un implant nucléique assurant la réparation du disque intervertébral endommagé.

L'appareil de nucléotomie suivant la présente invention comporte un porte outil pourvue de premier moyens de connexion assurant la mise en place d'un moteur électrique à entrainement rotatif, de second moyens de connexion pour la mise en place d'un outil de coupe à lame, des moyens d'entrainement assurant à l'outil de coupe des mouvements de rotation et de translation longitudinale et des moyens d'injection et d'aspiration permettant d'évacuer les débris du nucléus issus de la coupe lors de son aménagement à l'intérieur du disque intervertébral.

L'appareil de nucléotomie suivant la présente invention comporte des moyens de réglage limitant la course de déplacements en translation de l'outil de coupe à l'intérieur du disque intervertébral.

L'appareil de nucléotomie suivant la présente invention comporte des moyens de rétractation de l'outil de coupe avant son introduction dans le disque intervertébral.

L'appareil de nucléotomie suivant la présente invention comporte un outil de coupe qui est constitué d'une tige métallique souple enveloppée dans une gaine de protection en matière plastique, d'une liaison souple permettant de relier l'une des extrémité de la tige métallique à un couteau bilame réalisé en matériau super élastique, d'un élément de liaison reliant l'autre extrémité de la tige métallique au moyens d'entrainement du porte outil et une bague de connexion coopérant avec les second moyens de connexion pour l'immobilisation dudit outil de coupe sur ledit porte outil.

L'appareil de nucléotomie suivant la présente invention comporte un outil de coupe dont la bague de connexion coopère avec un mandrin de serrage permettant la mise en place et la retenue de la gaine de protection autour de la tige métallique souple.

L'appareil de nucléotomie suivant la présente invention comporte un outil de coupe dont la liaison souple de l'outil de coupe est constituée d'un câble de torsion fixé d'une part au couteau bilame et d'autre part à la tige métallique, ledit câble de torsion pouvant se déformer pour suivre des faibles rayons de courbure tout en assurant audit couteau bilame un entrainement en rotation à grande vitesse.

L'appareil de nucléotomie suivant la présente invention comporte un outil de coupe dont le câble de torsion est relié au couteau bilame par l'intermédiaire d'une liaison mécanique permettant d'une part le vissage dudit couteau bilame et d'autre part la fixation par soudure audit câble de torsion.

L'appareil de nucléotomie suivant la présente invention comporte un outil de coupe dont la liaison mécanique est constituée d'un manchon comportant à l'une de ses extrémités un alésage interne fileté destiné à coopérer avec une partie fileté du couteau bilame, tandis que l'autre extrémité du manchon se prolonge par épaulement de faible diamètre venant se loger à l'intérieur du câble de torsion pour être fixé par soudure à ce dernier.

L'appareil de nucléotomie suivant la présente invention comporte un porte outil qui est constitué d'un corps principal allongé et creux à l'intérieur duquel sont agencés et guidés les premier et second moyens de connexion et les moyens d'entrainement de l'outil de coupe, ledit corps principal étant solidaire perpendiculairement à l'une de ses extrémité d'une première poignée fixe à l'intérieur de laquelle est logé les moyens de réglage, tandis qu'une seconde poignée mobile chargée élastiquement pivote autour de la poignée fixe afin de pouvoir agir sur les moyens d'entrainement commandant les déplacements en translation longitudinale et en rotation de l'outil de coupe à lame.

L'appareil de nucléotomie suivant la présente invention comporte un porte outil dont les premiers moyens de connexion sont constitués d'un manchon cylindrique de liaison fixé et guidé à l'intérieur du corps principal, ledit manchon de liaison comportant un axe de liaison coopérant d'une part avec un axe de sortie du moteur électrique et d'autre part avec les moyens d'entrainement pour assurer l'entrainement en rotation de l'outil de coupe.

L'appareil de nucléotomie suivant la présente invention comporte un porte outil dont les second moyens de connexion sont constitués au niveau de l'extrémité libre du corps principal du porte outil de deux encoches diamétralement opposées et destinées à recevoir respectivement par encliquetage des languettes de verrouillage réalisées dans la bague de connexion de l'outil de coupe.

L'appareil de nucléotomie suivant la présente invention comporte un porte outil dont les moyens d'entrainement sont constitués d'un manchon cylindrique de liaison guidé en translation à l'intérieur du corps principal et comportant d'une part dans sa partie interne un axe de transmission libre en rotation qui coopère avec l'axe de liaison des premiers moyens de connexion pour assurer l'entraînement en rotation de l'outil de coupe, et d'autre part sur sa périphérie externe et à l'opposée l'un de l'autre des doigts de guidage, traversant respectivement une rainure oblongue aménagée dans le corps principal du porte outil, lesdits doigts de guidage débouchant à l'extérieur du corps principal pour être logés chacun à l'intérieur d'une rainure oblongue réalisée respectivement dans les branches d'une fourche solidaire de la poigné mobile pour assurer les déplacement en translation de l'outil de coupe.

L'appareil de nucléotomie suivant la présente invention comporte un porte outil dont le manchon de liaison des moyens d'entrainement comporte dans le prolongement de son axe de transmission et à l'opposée des premiers moyens de connexion un alésage de transmission permettant la réception de l'élément de liaison de l'outil de coupe afin de transmettre à ce dernier les mouvements de rotation issu du moteur électrique.

L'appareil de nucléotomie suivant la présente invention comporte un porte outil dont les moyens de réglages sont constitués d'une vis filetée guidée en rotation à l'intérieur de la poignée fixe du porte outil et solidaire à l'une de ses extrémité d'une molette permettant de positionner un curseur au niveau de graduations gravées de part et d'autre d'une ouverture oblongue aménagées dans la partie supérieure de ladite poignée fixe, ladite vis coopère avec un levier relié à un axe de rotation autour duquel pivote la poignée mobile de sorte que l'entrainement en rotation de la vis permet de positionner le levier dans différentes position angulaire afin de pouvoir agir sur la course de pivotement de la poigné mobile et par conséquent de limiter les déplacements en translation des moyens d'entrainement.

L'appareil de nucléotomie suivant la présente invention comporte un porte outil dont les moyens de rétraction sont constitués d'une gâchette guidée dans ses pivotements sur le corps principal de porte outil de manière à pouvoir déplacer un levier solidaire de ladite gâchette et dont l'extrémité libre est logée à l'intérieur d'un alésage ménagé dans ledit corps principal.

L'appareil de nucléotomie suivant la présente invention comporte des moyens d'injection et d'aspiration qui sont constitués d'un circuit d'injection dans lequel circule du sérum physiologique pénétrant à l'intérieur de l'espace nucléique Es en cours de formation et d'un circuit d'aspiration permettant la récupération du sérum physiologique et des débris produits par le couteau bilame de l'outil de coupe.

L'appareil de nucléotomie suivant la présente invention comporte des moyens d'injection et d'aspiration dont le circuit d'injection est raccordé à une pompe d'injection alimenté en sérum physiologique tandis que le circuit d'aspiration est connecté à une pompe d'aspiration comportant un réservoir pour la récupération du sérum physiologique et des débris.

L'appareil de nucléotomie suivant la présente invention comporte un moteur électrique à entrainement rotatif et des moyens d'injection et d'aspiration qui sont reliés à un boitier de commande piloté par l'utilisateur au moyen de pédales de commande.

L'appareil de nucléotomie suivant la présente invention comporte des moyens d'injection et d'aspiration qui sont constitués d'un connecteur de liaison venant se fixer d'une part sur l'extrémité d'un tube articulé préalablement implanté dans le corps de la vertèbre Va correspondante et d'autre part sur le corps principal du porte outil.

L'appareil de nucléotomie suivant la présente invention comporte un connecteur de liaison dont les moyens de fixation sont constitués d'une part d'un taquet venant coopérer avec la tête de fixation du tube articulé et d'autre part d'une pince articulée venant s'encliqueter autour du corps principal du porte outil.

L'appareil de nucléotomie suivant la présente invention comporte un connecteur de liaison comprenant des prises de raccordement pour la fixation des circuits d'injection et d'aspiration.

La description qui va suivre en regard des dessins annexés, donnés à titre d'exemples non limitatifs permettra de mieux comprendre l'invention, les caractéristiques qu'elle présente, et les avantages qu'elle est susceptible de procurer :
Figures 1 à 3 sont des vues schématiques en perspective illustrant l'appareil de nucléotomie suivant la présente invention
Figure 4 est une vue en coupe représentant un segment rachidien dans lequel est ménagé à l'intérieur du disque intervertébral Di un espace nucléique Es au moyen de l'appareil de nucléotomie suivant la présente invention.
Figures 5 à 8 sont des vues en perspective montrant le porte outil en forme de pistolet de l'appareil de nucléotomie sur lequel est agencé un outil de coupe à lame et des moyens d'entrainement suivant la présente invention.
Figures 9 et 10 sont des vues en coupe représentant le porte outil en forme de pistolet de l'appareil de nucléotomie sur lequel est agencé un outil de coupe à lame et des moyens d'entrainement suivant la présente invention.
Figures 11 à 13 sont des vues illustrant le connecteur de liaison assurant la fixation du corps principal en forme de pistolet sur un tube articulé guidé à l'intérieur du corps vertébral d'un segment rachidien.
Figures 14 à 16 sont des vues montrant l'outil de coupe et plus particulièrement l'agencement de la liaison souple entre le couteau bilame et la tige métallique suivant la présente.
Figures 17 à 20 sont des vue représentant les différentes étapes d'assemblage du porte outil en forme de pistolet et de l'outil de coupe à lame de l'appareil de nucléotomie suivant la présente invention.

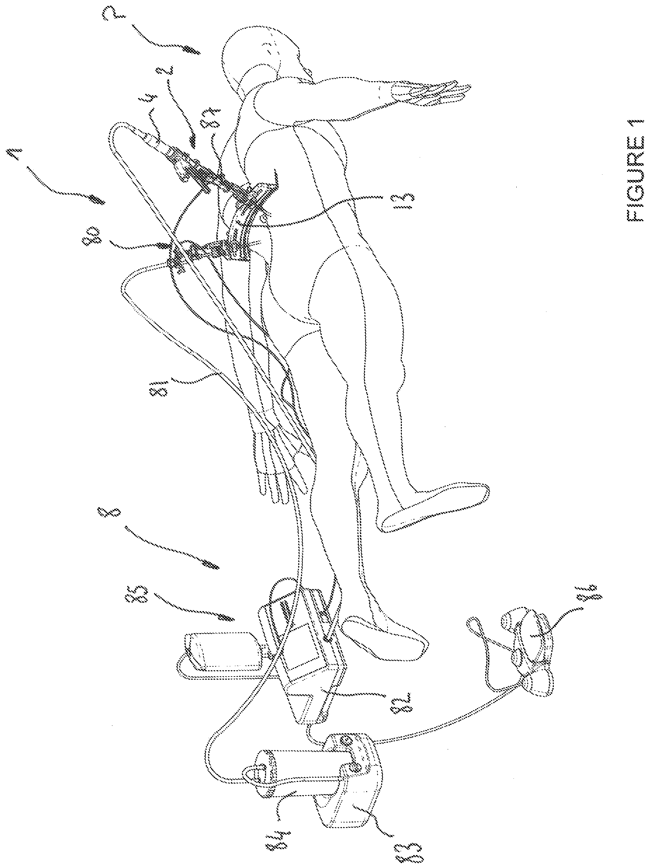

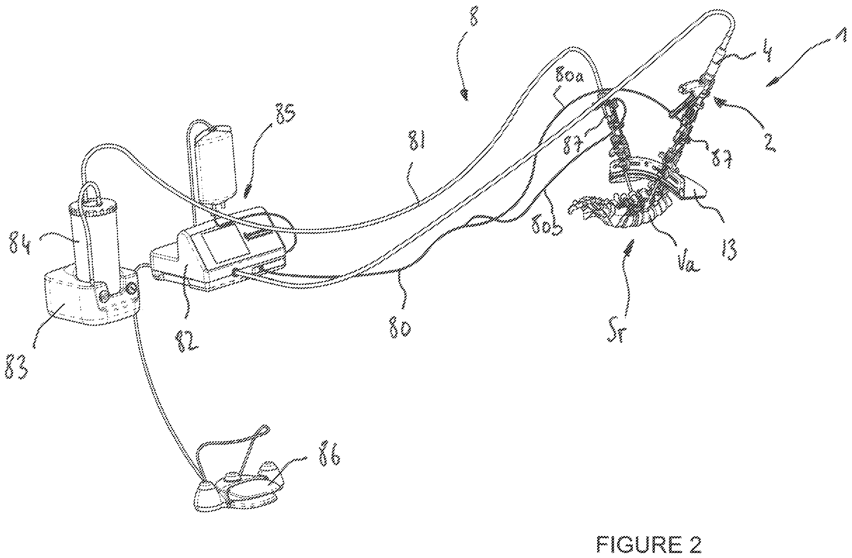

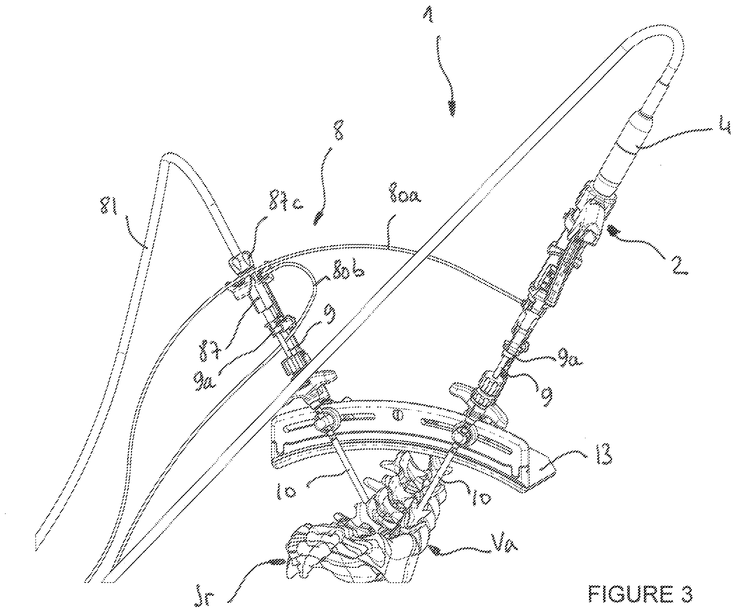

On a montré en figures 1 à 3 un appareil de nucléotomie 1 suivant la présente invention permettant, à partir de perçages Pr préalablement réalisés, suivant une voie d'abord trans-osseuse, dans le corps d'une vertèbre Va d'un segment rachidien Sr d'un patient P, la réalisation dans un disque intervertébral Di d'un espace nucléique Es.

L'appareil de nucléotomie 1 comprend un support de réglage angulaire 13 fixé sur la partie externe du corps du patient P et sur lequel est positionné et fixé deux canules 10 ancrées dans le corps de la vertèbre Va correspondante du segment rachidien Sr.

Les deux canules 10 coopèrent respectivement avec un tube articulée 9 comprenant une tête de fixation 9a permettant par l'intermédiaire de connecteur de liaison 87 la mise en place et la fixation d'un porte outil en forme de pistolet 2 comprenant des moyens de connexion 5 et des moyens d'entrainement 7 assurant respectivement la mise en place et l'entrainement en rotation et translation longitudinale d'un outil de coupe 6.

L'appareil de nucléotomie 1 comprend des moyens d'injection et d'aspiration 8 permettant d'évacuer les débris du nucléus issus de la coupe lors de son aménagement à l'intérieur du disque intervertébral Di.

Les moyens d'injection et d'aspiration 8 sont constitués d'un circuit d'injection 80 dans lequel circule du sérum physiologique pénétrant à l'intérieur de l'espace nucléique Es en cours de formation et d'un circuit d'aspiration 81 permettant la récupération du sérum physiologique et des débris produits par le couteau bilame 6a de l'outil de coupe 6.

Le circuit d'injection 80 est raccordé à une pompe d'injection 82 alimenté en sérum physiologique, tandis que le circuit d'aspiration 81 est connecté à une pompe d'aspiration 83 comportant un réservoir 84 pour la récupération du sérum physiologique et des débris produits par le couteau bilame 6a de l'outil de coupe 6.

Les moyens d'injection et d'aspiration 8 sont reliés à un boitier de commande 85 piloté par l'utilisateur au moyen de pédales de commande 86.

Les moyens d'injection et d'aspiration 8 comportent un premier et second connecteur de liaison 87 venant se fixer respectivement sur la tête de fixation 9a du premier et second tube articulé 9 introduit à l'intérieur de la première et seconde canule 10 préalablement ancrée dans le corps de la vertèbre Va correspondante du segment rachidien Sr.

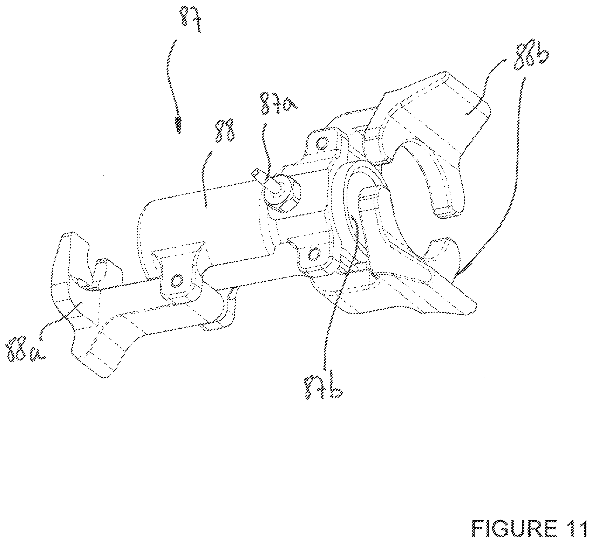

On a représenté en figures 11 à 13 le connecteur de liaison 87 qui est constitué d'un manchon cylindrique creux 88 comportant des moyens de fixation qui sont constitués d'une part d'un taquet 88**a** venant coopérer avec la tête de fixation 9**a** du tube articulé 9 correspondant et d'autre part d'une pince articulée 88**b** venant s'encliqueter autour du corps principal 2**a** du porte outil 2.

Les moyens de fixation sont également constitués à l'opposé du taquet 88**a** d'une pince articulé 88**b** comportant deux mâchoires prolongeant le manchon cylindrique et venant en position de verrouillage s'encliqueter autour du porte outil en forme de pistolet 2.

Le manchon cylindrique formant le connecteur de liaison 87 comporte une première prise de raccordement 87**a** permettant le branchement des tuyaux d'injection 80**a**, 80**b** provenant d'une séparation en Y du circuit d'injection 80 des moyens d'injection et d'aspiration 8.

Le manchon cylindrique formant le connecteur de liaison 87 comporte une seconde prise de raccordement 87**b** permettant soit le branchement par l'intermédiaire d'un bouchon de connexion 87**c** du circuit d'aspiration 81 des moyens d'injection et d'aspiration 8 soit la mise en place par l'intermédiaire d'une bague de connexion 6**e** de l'outil de coupe 6 agencé sur le corps principal 2**a** du porte outil 2.

Les prises de raccordement 87**a**, 87**b** sont positionnés sur le manchon cylindrique suivant des directions diamétralement opposées afin de déboucher respectivement à des niveaux différents l'intérieur du tube articulé 9 sur lequel et fixé le connecteur de liaison 87.

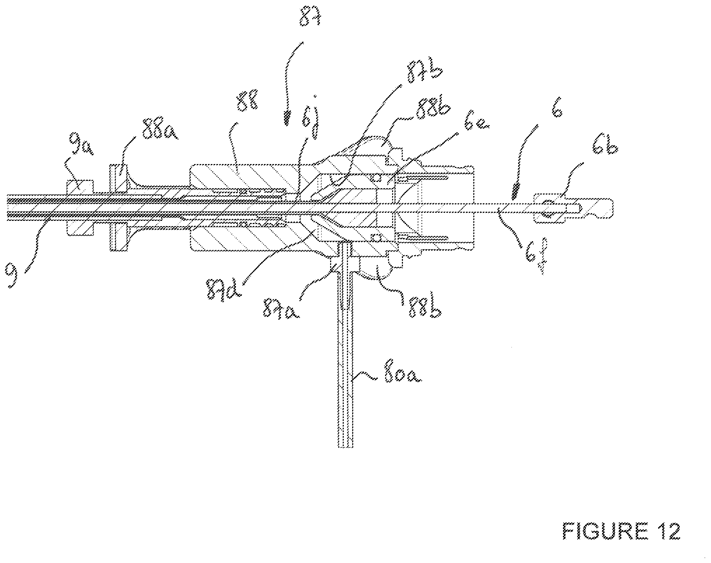

En figure 12, on a illustré le connecteur de liaison 87 agencé sur le premier tube articulé 9 et dont la première prise de raccordement 87**a** coopère, par exemple, avec le tuyau 80**a** du circuit d'injection 80, tandis que la seconde prise de raccordement 87**b** coopère de manière étanche avec la bague de connexion 6**e** de l'outil de coupe 6.

Dans cette configuration, on note que la première prise de raccordement 87**a** débouche dans une espace libre 87**d** formé entre la face interne de la seconde prise de raccordement 87**b** et la face externe de la bague de connexion 6**e** de l'outil de coupe 6.

Ainsi, le sérum physiologique provenant de la pompe d'injection 82 est introduit dans le connecteur de liaison 87 via le tuyau 80**a** et la première prise de raccordement 87**a** afin de traverser l'espace libre 87**d** et circuler à l'intérieur du premier tube articulé 9 et plus particulièrement entre la face interne d'un alésage 9**b** et la face externe d'une gaine 6**j** de l'outil de coupe 6 pour venir déboucher à l'intérieur de l'espace nucléique Es en cours de formation.

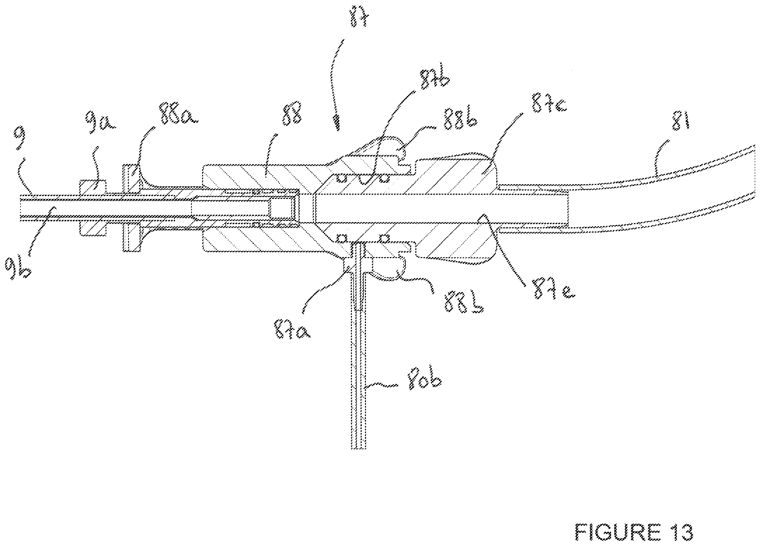

En figure 13, on a représenté le connecteur de liaison 87 agencé sur le second tube articulé 9 et dont la première prise de raccordement 87**a** coopère, par exemple, avec le tuyau 80**b** du circuit d'injection 80, tandis que la seconde prise de raccordement 87**b** coopère de manière étanche avec le bouchon de connexion 87**c** dont l'alésage interne 87**e** est relié au circuit d'aspiration 81 des moyens d'injection et d'aspiration 8.

Dans cette configuration, on constate que la première prise de raccordement 87**a** est obstruer par le bouchon de connexion 87**c** de manière étanche afin d'empêcher la circulation du sérum physiologique, provenant de la pompe d'injection 82, à l'intérieur du second tube articulé 9.

Par contre, on note que l'alésage interne 87**e** du bouchon de connexion 87**c** coopère à l'opposé du circuit d'aspiration 81 avec l'alésage interne 9**b** du second tube 9 afin de pouvoir aspirer le sérum physiologique se trouvant à l'intérieur de l'espace nucléique Es en cours de formation.

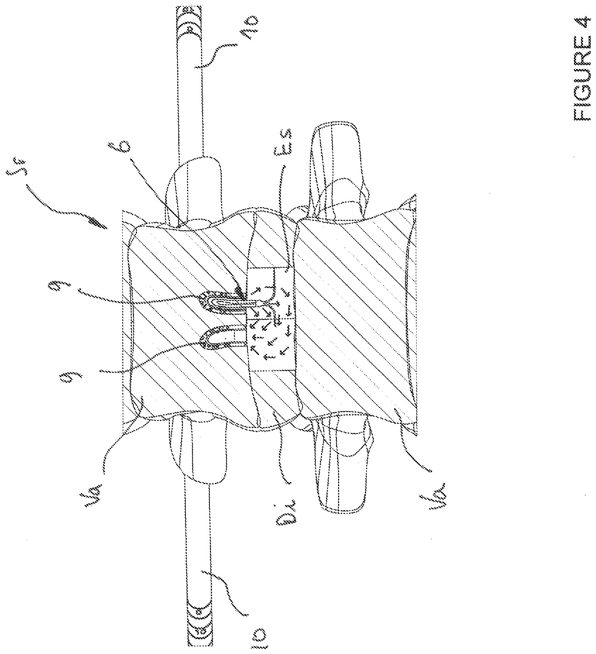

On note que cet agencement particulier permet une circulation du sérum physiologique introduit par la pompe d'injection 82 dans le circuit d'injection 80**a** du premier connecteur de liaison 87 et la récupération, après avoir traversé l'espace nucléique Es, dudit sérum physiologique et les débris produits par le couteau bilame de l'outil de coupe 6 par la pompe d'aspiration 83 raccordé au circuit d'aspiration 81 du second connecteur de liaison 87 (figure 4).

On constate que le sérum physiologique circule est boucle fermée de manière à entrée par le premier connecteur de liaison 87 agencé sur le premier tube articulé 9 et portant le porte outil en forme de pistolet 2 pour sortir par le second connecteur de liaison 87 monté sur le second tube articulé 9 et qui a été préalablement obstrué par un bouchon 87**c**.

On note que le chirurgien doit procéder de manière inverse à ce qui a été décrit ci-dessus lorsque le porte outil en forme de pistolet 2 est fixé sur le second connecteur de liaison 87.

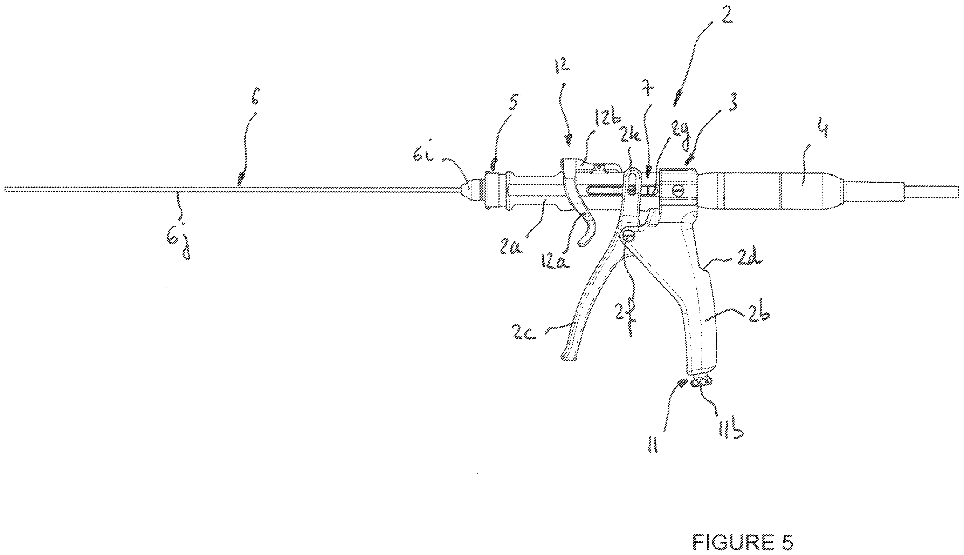

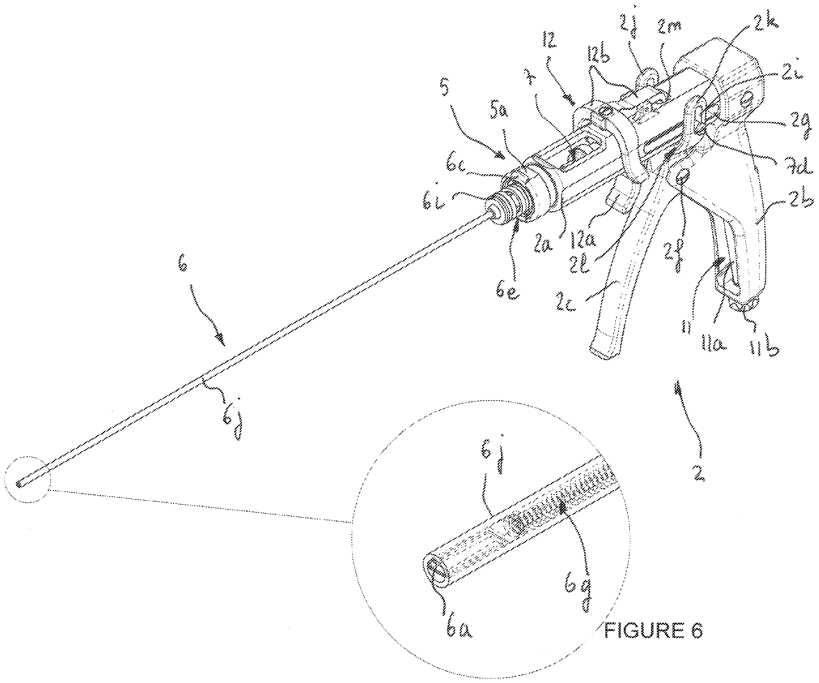

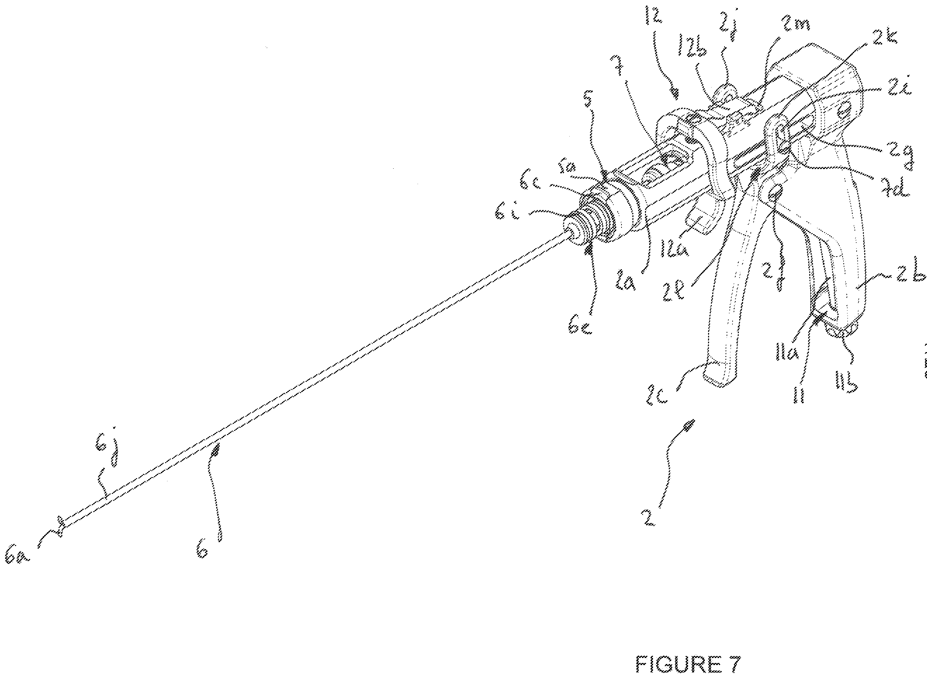

On a montré en figures 5 à 10 le porte outil en forme de pistolet 2 de l'appareil de nucléotomie 1 permettant de réaliser par l'intermédiaire d'un outil de coupe 6 l'espace nucléique Es à l'intérieur du disque intervertébral Di.

Le porte outil en forme de pistolet 2 est constitué d'un corps principal 2**a** allongé et creux à l'intérieur duquel sont agencés et guidés des premier et second moyens de connexion 3 et 5 et les moyens d'entrainement 7 de l'outil de coupe 6.

Le corps principal 2**a** du porte outil en forme de pistolet 2 est solidaire perpendiculairement à l'une de ses extrémité d'une première poignée fixe 2**b** à l'intérieur de laquelle est logé des moyens de réglage 11, tandis qu'une seconde poignée mobile 2**c** chargée élastiquement pivote autour de la poignée fixe 2**b** afin de pouvoir agir sur les moyens d'entrainement 7 commandant les déplacements en translation longitudinale et en rotation de l'outil de coupe à lame 6.

Les premiers moyens de connexion 3 logés à l'intérieur du corps principal 2**a** du porte outil en forme de pistolet 2 permettent la mise en place et la fixation d'un moteur électrique 4 à entrainement rotatif assurant l'entrainement en rotation par l'intermédiaire des moyens d'entrainement 7 de l'outil de coupe 6.

Les premiers moyens de connexion 3 sont constitués d'un manchon cylindrique de liaison 3**a** fixé à l'intérieur du corps principal 2**a** du porte outil en forme de pistolet 2 et au dessus de la poignée fixe 2**b**.

Le manchon de liaison 3**a** permet de recevoir par encliquetage le moteur électrique 4 de manière que son axe de sortie 4**a** coopère avec un axe de liaison 3**b** guidé axialement à l'intérieur du corps principal 2**a** du porte outil en forme de pistolet 2. L'axe de liaison 3**b** coopère avec les moyens d'entrainement 7 pour assurer l'entrainement en rotation de l'outil de coupe 6.

Le moteur électrique 4 à entrainement rotatif est relié par l'intermédiaire d'un cordon électrique au boitier de commande 85 qui est piloté par l'utilisateur au moyen des pédales de commande 86.

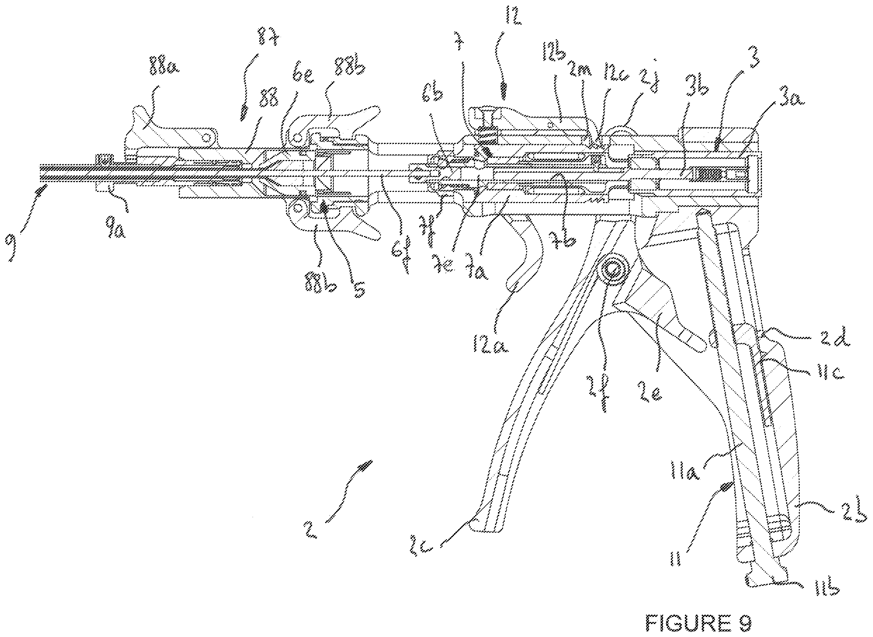

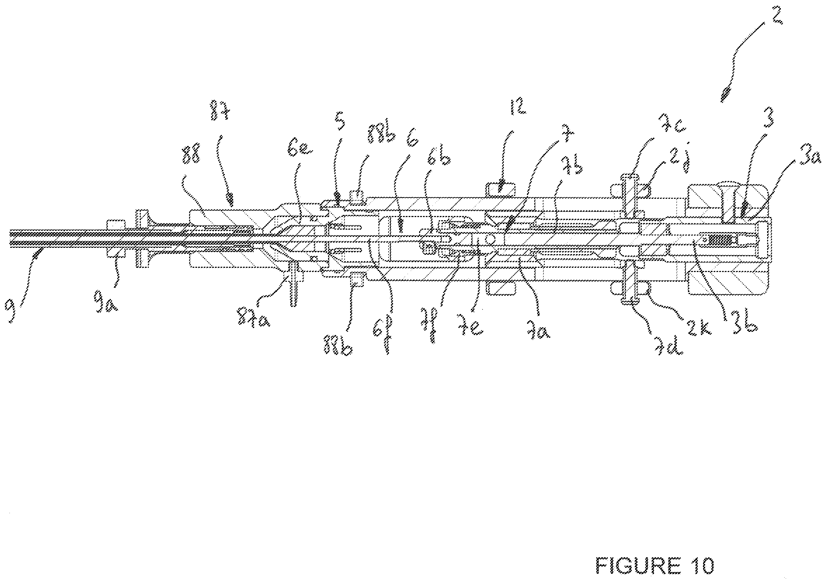

En figures 9 et 10 on a illustré les moyens d'entrainement 7 qui sont constitués d'un manchon cylindrique de liaison 7**a** guidé en translation à l'intérieur du corps principal 2**a** du porte outil en forme de pistolet 2.

Le manchon de liaison 7**a** comporte dans sa partie interne un axe de transmission 7**b** libre en rotation et qui coopère avec l'axe de liaison 3**b** des premiers moyens de connexion 3
Le manchon de liaison 7**a** comporte sur sa périphérie externe et à l'opposée l'un de l'autre des doigts de guidage 7**c**, 7**d**, traversant respectivement une rainure oblongue 2**g** aménagée dans le corps principal 2**a** du porte outil en forme de pistolet 2.

Les doigts de guidage 7**c**, 7**d**, du manchon de liaison 7**a** débouchent à l'extérieur du corps principal 2**a** pour être logés chacun à l'intérieur d'une rainure oblongue 2**h**, 2**i** réalisée respectivement dans les branches 2**j** et 2**k** d'une fourche 2**l** solidaire de la poigné mobile 2**c**.

Le manchon de liaison 7**a** comporte dans le prolongement de son axe de transmission 7**b** et à l'opposée des premiers moyens de connexion 3 un alésage de transmission 7**e** permettant la réception d'un élément de liaison 6**b** solidaire de l'outil de coupe 6 afin de transmettre à ce dernier les mouvements de rotation issu du moteur électrique 4.

On note que lorsque la poigné mobile 2**c** est activée, cette dernière permet par l'intermédiaire de ses branches 2**j** et 2**k** s'étendant au dessus de l'axe de rotation 2**f** de déplacer le manchon de liaison 7**a** solidaire de son axe de transmission 7**b** en translation à l'intérieur du corps principal 2**a** pour assurer simultanément les déplacements en translation et en rotation de l'outil de coupe 6 fixé audit corps principal 2**a** par l'intermédiaire des seconds moyens de connexion 5.

Les second moyens de connexion 5 et les moyens d'entrainement 7 guidés à l'intérieur du corps principal 2**a** du porte outil en forme de pistolet 2 assurent respectivement la mise en place et l'entrainement en rotation et en translation longitudinale de l'outil de coupe 6.

Les seconds moyens de connexion 5 sont constitués au niveau de l'extrémité libre du corps principal 2**a** du porte outil en forme de pistolet 2 de deux encoches 5**a**, 5**b** diamétralement opposées et destinées à recevoir respectivement par encliquetage les languettes de verrouillage 6**c**, 6**d** réalisées dans une bague de connexion 6**e** de l'outil de coupe 6.

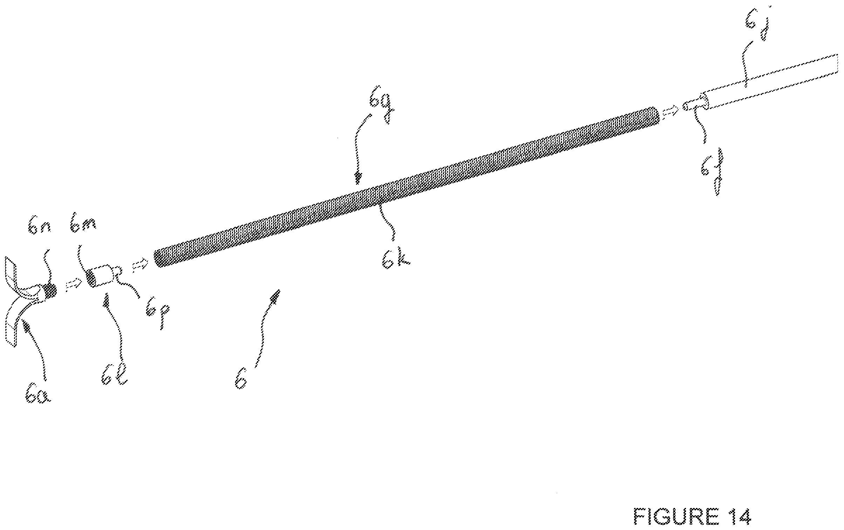

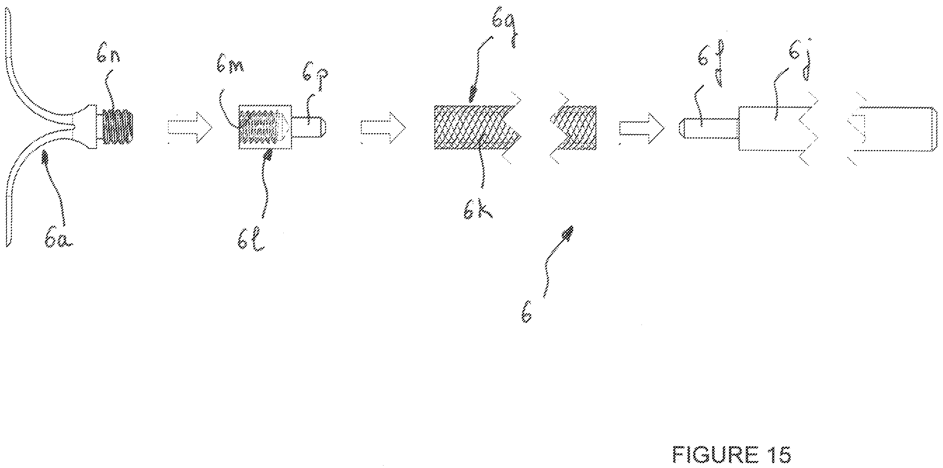

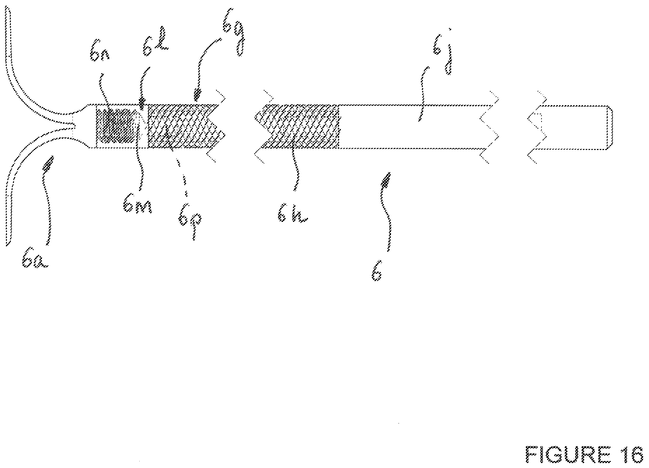

On a montré en figures 14 à 16 l'outil de coupe 6 est constitué d'une tige métallique souple 6**f** qui est solidaire à l'une de ses extrémité par l'intermédiaire d'une liaison souple 6**g** du couteau bilame 6**a** réalisé en matériau déformable, tandis que l'autre extrémité reçoit après la mise en place de la bague de connexion 6**e** l'élément de liaison 6b qui est immobilisé en translation sur ladite tige métallique par une vis de serrage 6**h**.

La bague de connexion 6**e** coopère avec un mandrin de serrage 6**i** permettant la mise en place et la retenue d'une gaine de protection 6**j** réalisée en matière plastique et venant envelopper la tige métallique souple 6**f** et la liaison souple 6**g** portant le couteau bilame 6**a**.

La liaison souple 6**g** permettant de relier l'une des extrémités de la tige métallique au couteau bilame 6**a** est réalisée dans un matériau déformable afin que l'outil de coupe 6 puisse être déformé et suivre de faible rayons de courbure.

La liaison souple 6**g** de l'outil de coupe 6 est constituée d'un câble de torsion 6**k** fixé d'une part au couteau bilame 6**a** et d'autre part à la tige métallique 6**f**.

Le câble de torsion 6**k** peut se déformer pour suivre des faibles rayons de courbure tout en assurant audit couteau bilame 6**a** un entrainement en rotation à grande vitesse.

Le câble de torsion 6**k** est relié au couteau bilame 6**a** par l'intermédiaire d'une liaison mécanique 6**l** permettant d'une part le vissage dudit couteau bilame 6**a** et d'autre part la fixation par soudure dudit câble de torsion 6**k**.

La liaison mécanique 6**l** est constituée d'un manchon comportant à l'une de ses extrémités un alésage interne fileté 6**m** destiné à coopérer avec la partie fileté 6n du couteau bilame 6**a**, tandis que l'autre extrémité du manchon se prolonge par épaulement 6**p** de faible diamètre venant se loger à l'intérieur du câble de torsion 6**k** pour être fixé à ce dernier par soudure.

La fixation par liaison fileté entre l'alésage interne 6**m** et la partie filetée 6**n** du couteau bilame 6**a** est complétée par une liaison par soudure empêchant tout risque de desserrage des deux parties filetées.

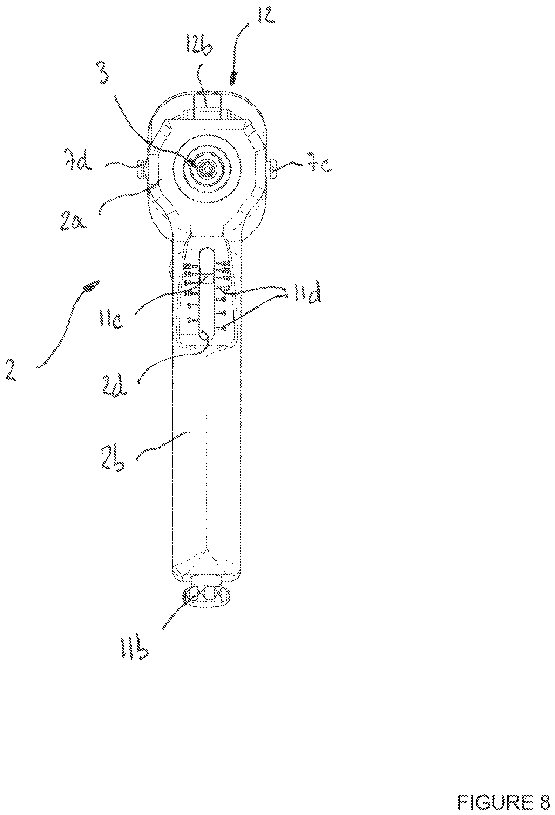

Le câble de torsion 6**k** est fixé à l'opposé du couteau bilame 6**a** à la tige métallique 6**f**. La tige métallique 6**f** présente un faible diamètre externe de manière à venir se loger à l'intérieur du câble de torsion 6**k** et être fixé à ce dernier par soudure. L'appareil de nucléotomie 1 comporte des moyens de réglages 11 qui sont constitués d'une vis filetée 11**a** guidée en rotation à l'intérieur de la poignée fixe 2**b** et solidaire à l'une de ses extrémité d'une molette 11**b** permettant de positionner un curseur 11**c** au niveau de graduations 11**d** gravées de part et d'autre d'une ouverture oblongue 2**d** aménagées dans la partie supérieure de la poignée fixe 2**b** (figures 8 et 9).

La vis 11**a** coopère avec un levier 2**e** relié à un axe de rotation 2**f** autour duquel pivote la poignée mobile 2**c**.

L'entrainement en rotation de la vis 11**a** permet de positionner le levier 2**e** dans différentes position angulaire afin de pouvoir agir sur la course de pivotement de la poigné mobile 2**c** et par conséquent de limiter les déplacements en translation des moyens d'entrainement 7.

L'appareil de nucléotomie 1 comporte des moyens de rétraction 12 permettant de positionner le couteau bilame 6**a** du l'outil de coupe 6 à l'intérieur de la gaine de protection 6**j** prévu autour de la tige métallique 6**f** (figures 9 et 10).

En effet, les moyens de rétraction 12 permettent d'agir sur la tige métallique souple 6**f** en tirant sur cette dernière de manière que le couteau bilame 6**a** du fait de sa matière super élastique se déforme et pénètre à l'intérieur de la gaine de protection 6**j**.

Les moyens de rétraction 12 sont constitués d'une gâchette 12**a** guidée dans ses pivotement sur le corps principal 2**a** de porte outil 2 de manière à pouvoir déplacer un levier 12**b** solidaire de ladite gâchette 12**a** et dont l'extrémité libre 12**c** est logée à l'intérieur d'un alésage 2**m** ménagé dans ledit corps principal 2**a**. En position de repos l'extrémité libre 12**c** du levier 12**b** est en contact permanent avec le manchon de liaison 7**a** des moyens d'entrainement 7 auquel est connecté l'outil de coupe 6 limitant la course arrière dudit manchon, c'est-à-dire en direction des premiers moyens de connexion de l'appareil de nucléotomie 1.

Pour permettre l'introduction du couteau bilame 6**a** à l'intérieur de la gaine de protection 6**j**, le chirurgien doit :
- appuyer sur la gâchette 12**a** des moyens de rétraction 12 afin de libérer la course arrière du manchon de liaison 7**a** des moyens d'entrainement 7,
- tirer sur la poignée mobile 2**c** du porte outil 2 pour déplacer les moyens d'entrainement 7 vers l'arrière dudit porte outil 2 entrainant de fait dans la même direction le manchon de liaison 7**a** auquel est connecté la tige métallique souple 6**f** de l'outil de coupe 6 permettant de contraindre le couteau bilame 6**a** afin que ce dernier pénètre à l'intérieur de la gaine de protection 6**j**.

Lorsque le couteau bilame 6**a** de l'outil de coupe 6 monté sur le porte outil 2 de l'appareil de nucléotomie 1 est en position rétracté le chirurgien peut introduire l'outil de coupe 6 à l'intérieur de la canule 9 afin d'amener ledit outil de coupe au niveau du disque intervertébral Di dans lequel doit être réalisé l'espace nucléique Es.

### Fonctionnement de l'appareil de Nucléotomie

### • Préparation de l'appareil de Nucléotomie

Le chirurgien procède à la mise en place et au verrouillage des deux tubes articulés 9 à l'intérieur de la canule 10 correspondante, ladite canule étant préalablement fixée et ancrée dans le corps de la vertèbre Va du segment rachidien Sr afin de diriger lesdites canules au dessus du disque intervertébral Di dans lequel doit être aménagé l'espace nucléique Es.

Il fixe sur chacun des premier et second tubes articulés 9 un premier et second connecteur de liaison 87 permettant la mise en place des tuyaux formant les circuits d'injection 80 et d'aspiration 81 reliant respectivement la pompe d'injection 82 et la pompe d'aspiration 83 des moyens d'injection et d'aspiration 8.

Le moteur électrique 4 et la pédale de commande 86 sont également connectés au boitier de commande 85 des moyens d'injection et d'aspiration 8.

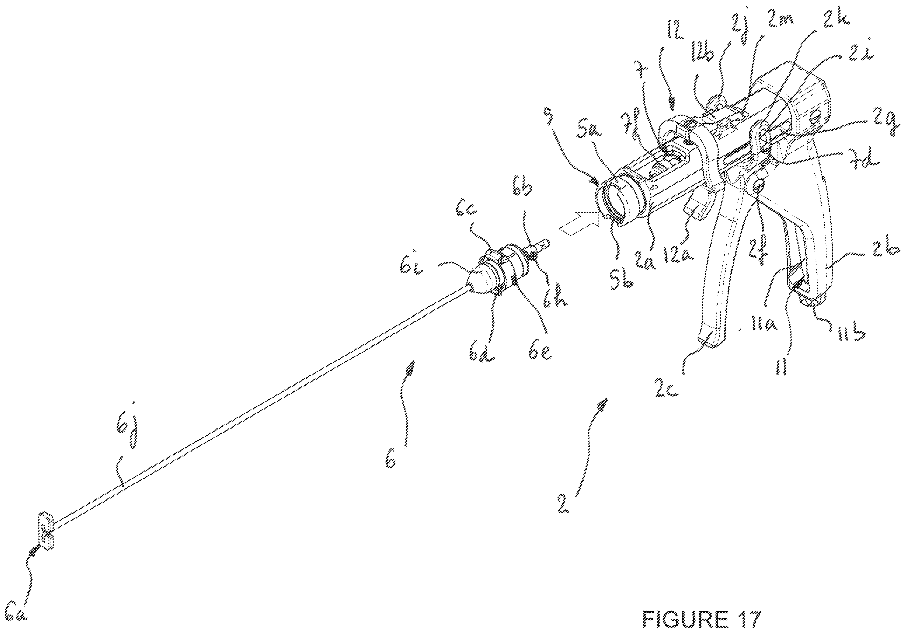

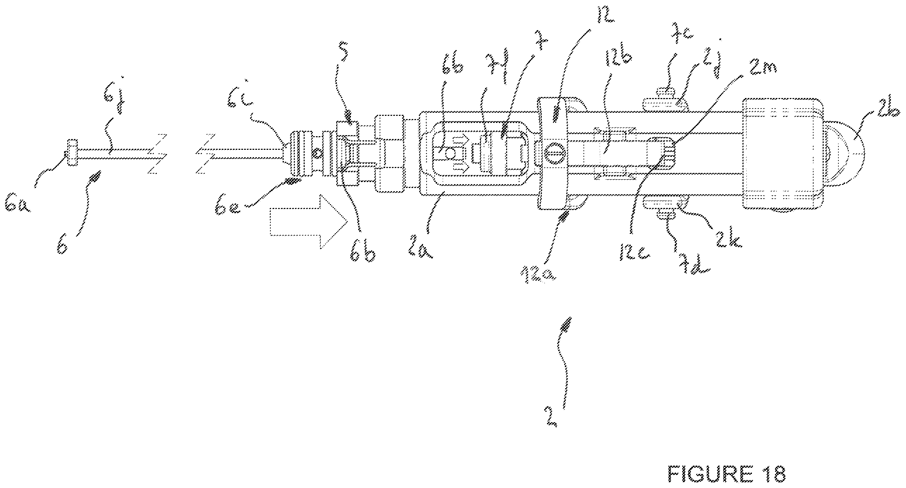

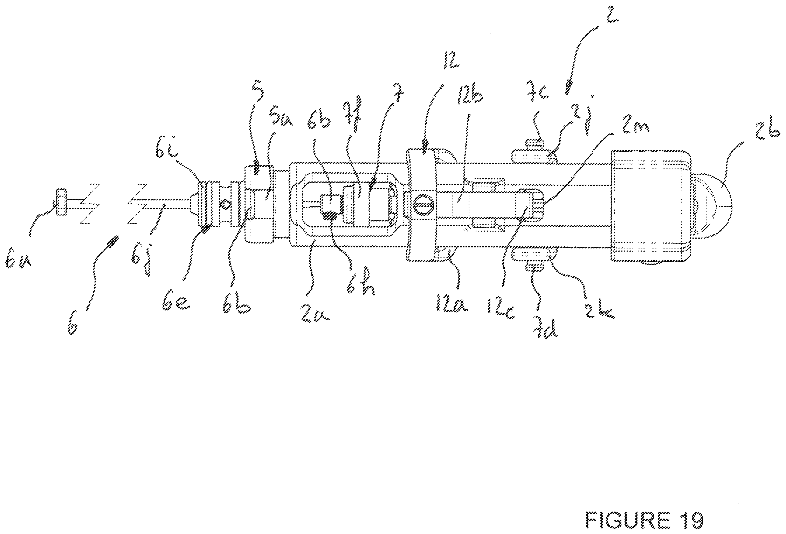

Le chirurgien choisit dans un jeu d'outil de coupe un premier outil de coupe 6 dont le couteau bilame 6**a** présente un diamètre de coupe de 10 millimètres. Le jeu d'outil comporte deux autres outils de coupe 6 dont le couteau bilame 6**a** présente respectivement un diamètre de coupe de 14 et 18 millimètres (figures 17 à 19).

Pour cela le chirurgien appui sur la bague externe 7**f** des moyens d'entrainement 7 afin de pouvoir insérer l'élément de liaison 6**b** à l'intérieur de l'alésage de transmission 7**e** desdits moyens d'entraînement. Simultanément à l'introduction de l'élément de liaison 6**b** la bague de connexion 6**e** de l'outil de coupe 6 est connectée au second moyen de connexion 5 que comporte le corps principal 2**a** du porte outil 2.

Le verrouillage de l'outil de coupe 6 sur le porte outil 2 est définitivement obtenue lorsque le chirurgien relâche la bague externe 7**f** des moyens d'entrainement 7.

Le chirurgien doit procéder ensuite à la protection du couteau bilame 6**a** de l'outil coupe 6 avant son introduction dans le premier tube articulé 9. Pour cela il appui sur la gâchette 12**a** des moyens de rétraction 12, puis il tire simultanément sur la poignée mobile 2**c** du porte outil 2 de manière à déplacer la tige métallique 6**f** et le couteau bilame 6**a** à l'intérieur de la gaine de protection 6**j**.

La gâchette 12**a** permet au moyen de son levier 12**b** de bloquer en translation les moyens d'entrainement 7 et de maintenir le couteau bilame 6**a** à l'intérieur de ladite gaine de protection 6**j**, lorsque le chirurgien relâche ladite gâchette 12**a** et ladite poignée mobile 2**c**.

Avant l'introduction de l'outil de coupe 6 dans le premier tube articulé 9, le chirurgien procède au réglage de la course de ce dernier en fonction de la hauteur du disque intervertébral Di. Pour cela, il tourne la molette 11**b** des moyens de réglage 11 afin de positionner le curseur 11**c** sur la graduation 11**d** correspondant à la hauteur dudit disque intervertébral Di.

Les moyens de réglage 11 permettent d'agir sur le débattement angulaire de la poignée mobile 2**c** limitant cette dernière et donc le mouvement en translation des moyens d'entraînement 7.

Enfin, le chirurgien monte le moteur électrique 4 sur le corps principal 2**a** du porte outil 2 de manière que l'axe de sortie 4**a** dudit moteur coopère avec l'axe de liaison 3**a** des premiers moyens de connexion dudit porte outil 2.

### • Réalisation de la nucléotomie

Le chirurgien introduit l'outil de coupe 6 assemblé au porte outil 2 à l'intérieur du premier tube articulé 9 préalablement positionné à l'intérieur de la première canule 10. L'outil de coupe 6 est totalement introduit jusqu'à ce que le porte outil 2 vienne en appui contre le connecteur de liaison 87 préalablement fixé au premier tube articulé 9 au moyen du taquet 88**a**. Le chirurgien procède à la fixation du porte outil 2 sur le connecteur de liaison 87 en fermant les mâchoires de la pince articulée 88**b** afin que ces dernières viennent coopérer avec une rainure périphérique circulaire 2**n** ménagée dans le corps principal 2**a**.

Le chirurgien fixe sur l'autre connecteur de liaison 87 monté sur le second tube articulé 9 un bouchon 87**c** permettant au circuit d'injection 80 et d'aspiration 81 de fonctionner en circuit fermé.

Le chirurgien procède à la réalisation à l'intérieur du disque intervertébral Di de l'espace nucléique Es par l'introduction progressive du couteau bilame 6**a** de l'outil 6.

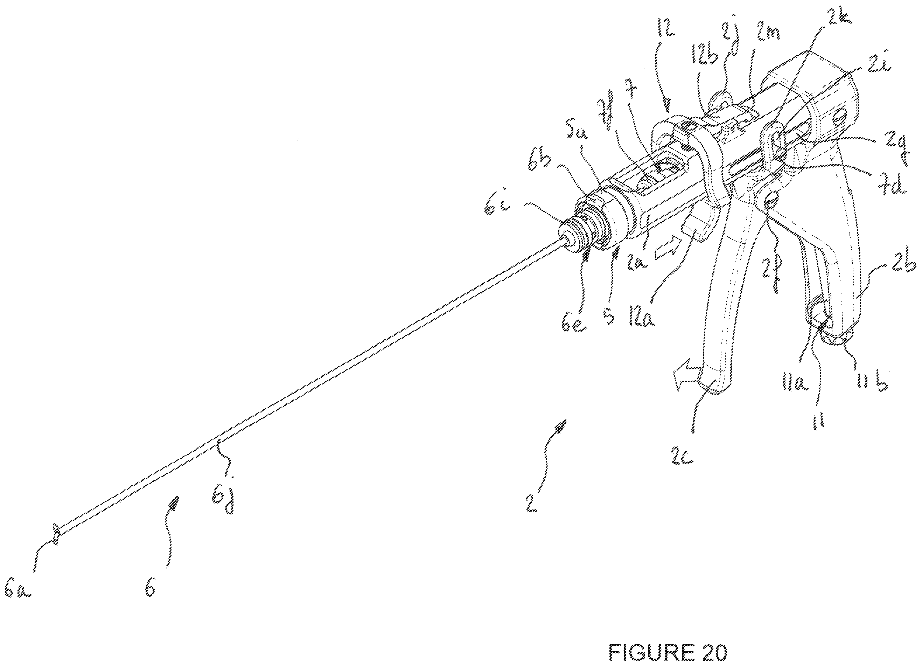

Pour cela, il appui simultanément sur la gâchette 12**a** et sur la poignée mobile 2**c** du porte outil 2 pour libérer les moyens d'entrainement 7 et déployer le couteau bilame 6**a** à l'extérieur de la gaine de protection 6**j** (figure 20).

Il choisit sur le boitier de commande 85 le protocole de nucléotomie déterminant la vitesse de rotation et le couple de l'outil de coupe 6 et la mise en route des pompes d'injection 82 et d'aspiration 83.

Ensuite, il appuie sur la pédale de commande 86 pour commander le moteur électrique 4 et la rotation de l'outil de coupe 6.

Il procède à des déplacements successifs « Appuyer et Relâcher » de la poignée mobile 2**c** du porte outil 2 pour réaliser des mouvements progressifs de va et vient de l'outil de coupe 6, afin que le couteau bilame 6**a** progresse à l'intérieur du disque intervertébral Di et sur la profondeur préalablement réglée par l'intermédiaire des moyens de réglage 11.

Lorsque la nucléotomie avec l'outil de coupe 6 de diamètre 10 millimètres est terminée, le chirurgien relâche la pédale de commande 86, rétracte le couteau bilame 6**a** à l'intérieur de la gaine 6**j**, et libère le porte outil 2 du premier connecteur de liaison 87 et extrait ledit porte outil 2 du premier tube articulé 9.

Ensuite, le chirurgien dévisse le bouchon 87**c** du second connecteur 87 agencé sur le second tube articulé 9 pour pouvoir insérer le porte outil 2 muni de son outil de coupe 6 de diamètre 10 millimètre à l'intérieur dudit tube pour venir en contact avec le disque intervertébral Di.

Il visse le bouchon 87**c** sur le premier connecteur 87 agencé sur le premier tube articulé 9 dont il vient de démonter le porte outil 2 et introduit le porte outil 2 et son outil de coupe 6 de diamètre 10 millimètres à l'intérieur du second tube articulé 9.

Il positionne et met en marche les pompes d'injection 82 et d'aspiration 83 pour que le liquide physiologique circule dans le bon sens dans les circuits d'injections 80 et d'aspiration 81 et dans l'espace nucléique Es encours de réalisation.

Le chirurgien procède aux mêmes manipulations du porte outil 2 que celles décrites aux étapes précédentes ci-dessus pour la réalisation de la nucléotomie.

Lorsque la nucléotomie avec l'outil de coupe 6 de diamètre 10 millimètres est terminée, le chirurgien relâche la pédale de commande 86, rétracte le couteau bilame 6**a** à l'intérieur de la gaine 6**j**, et libère le porte outil 2 du second connecteur de liaison 87 et extrait ledit porte outil 2 du second tube articulé 9.

Ensuite le chirurgien prend un autre porte outil 2 solidaire d'un outil de coupe 6 dont le diamètre de coupe est de 14 millimètres, l'introduit dans le second tube articulé 9 pour le fixé sur le second connecteur de liaison 87 pour procéder aux mêmes opérations que décrites précédemment pour le porte outil 2 solidaire de l'outil de coupe 6 dont le diamètre de coupe est de 10 millimètres.

Enfin le chirurgien prend un autre porte outil 2 solidaire d'un outil de coupe 6 dont le diamètre de coupe est de 18 millimètre l'introduit dans le premier tube articulé 9 pour le fixé sur le premier connecteur de liaison 87 pour procéder aux mêmes opérations que décrites précédemment pour le porte outil 2 solidaire de l'outil de coupe 6 dont le diamètre de coupe est de 10 millimètres.

Lorsque la nucléotomie avec l'outil de coupe 6 de diamètre 18 millimètres est terminée, le chirurgien relâche la pédale de commande 86, rétracte le couteau bilame 6**a** à l'intérieur de la gaine 6**j**, et libère le porte outil 2 du second connecteur de liaison 87 et extrait ledit porte outil 2 du second tube articulé 9.

La nucléotomie est terminée et l'espace nucléique Es réalisé à l'intérieur du disque intervertébral Di est près à recevoir un implant
Il doit d'ailleurs être entendu que la description qui précède n'a été donnée qu'à titre d'exemple et quelle ne limite nullement le domaine de l'invention dont on ne sortirait pas en remplaçant les détails d'exécution décrits par tout autre équivalent.

## Revendications

1. Appareil de nucléotomie permettant, à partir d'une voie d'abord trans-osseuse préalablement percée dans le corps d'une vertèbre Va, la réalisation dans un disque intervertébral Di d'un espace nucléique Es, qui comporte un porte outil (2) pourvue de premier moyens de connexion (3) assurant la mise en place d'un moteur électrique (4) à entrainement rotatif, de second moyens de connexion (5) pour la mise en place d'un outil de coupe à lame (6), des moyens d'entrainement (7) assurant à l'outil de coupe (6) des mouvements de rotation et de translation longitudinale, caractérisé en ce que" lesdits moyens d'entrainement (7) étant constitués d'un manchon cylindrique de liaison (7**a**) guidé en translation à l'intérieur du corps principal (2**a**) et comportant d'une part dans sa partie interne un axe de transmission (7**b**) libre en rotation qui coopère avec l'axe de liaison (3**b**) des premiers moyens de connexion (3) pour assurer l'entrainement en rotation de l'outil de coupe (6), et d'autre part sur sa périphérie externe et à l'opposée l'un de l'autre des doigts de guidage (7**c**, 7**d**), traversant respectivement une rainure oblongue (2**g**) aménagée dans le corps principal (2**a**) du porte outil (2) pour coopérer avec des branches (2**j** et 2**k**) d'une fourche (2**l**) solidaire d'une poigne mobile (2**c**) pour assurer les déplacements en translation de l'outil de coupe (6) et des moyens d'injection et d'aspiration (8) permettant d'évacuer les débris du nucléus issus de la coupe lors de son aménagement à l'intérieur du disque intervertébral.

2. Appareil de nucléotomie suivant la revendication 1, **caractérisé en ce qu'**il comporte des moyens de réglage (11) limitant la course de déplacements en translation de l'outil de coupe (6) à l'intérieur du disque intervertébral.

3. Appareil de nucléotomie suivant la revendication 1, **caractérisé en ce qu'**il comporte des moyens de rétractation (12) de l'outil de coupe avant son introduction dans le disque intervertébral.

4. Appareil de nucléotomie suivant la revendication 1, **caractérisé en ce que** l'outil de coupe (6) est constitué d'une tige métallique souple (6**f**) enveloppée dans une gaine de protection (6**j**) en matière plastique, d'une liaison souple (6**g**) permettant de relier l'une des extrémité de la tige métallique (6**f**) à un couteau bilame (6**a**) réalisé en matériau super élastique, d'un élément de liaison (6**b**) reliant l'autre extrémité de la tige métallique (6**f**) au moyens d'entrainement (7) du porte outil (2) et une bague de connexion (6**e**) coopérant avec les second moyens de connexion (5) pour l'immobilisation dudit outil de coupe (6) sur ledit porte outil (2).

5. Appareil de nucléotomie suivant la revendication 4, **caractérisé en ce que** la bague de connexion (6**e**) coopère avec un mandrin de serrage (6**i**) permettant la mise en place et la retenue de la gaine de protection (6**j**) autour de la tige métallique souple (6**f**).

6. Appareil de nucléotomie suivant la revendication 4, **caractérisé en ce que** la liaison souple (6**g**) de l'outil de coupe (6) est constituée d'un câble de torsion (6**k**) fixé d'une part au couteau bilame (6**a**) et d'autre part à la tige métallique (6**f**), ledit câble de torsion (6**k**) pouvant se déformer pour suivre des faibles rayons de courbure tout en assurant audit couteau bilame (6**a**) un entrainement en rotation à grande vitesse.

7. Appareil de nucléotomie suivant la revendication 6, **caractérisé en ce que** le câble de torsion (6**k**) est relié au couteau bilame (6**a**) par l'intermédiaire d'une liaison mécanique (6**l**) permettant d'une part le vissage dudit couteau bilame (6**a**) et d'autre part la fixation par soudure audit câble de torsion (6**k**).

8. Appareil de nucléotomie suivant la revendication 7, **caractérisé en ce que** la liaison mécanique (6**l**) est constituée d'un manchon comportant à l'une de ses extrémités un alésage interne fileté (6**m**) destiné à coopérer avec une partie fileté (6**n**) du couteau bilame (6**a**), tandis que l'autre extrémité du manchon se prolonge par épaulement (6**p**) de faible diamètre venant se loger à l'intérieur du câble de torsion (6**k**) pour être fixé par soudure à ce dernier.

9. Appareil de nucléotomie suivant la revendication 1, **caractérisé en ce que** le porte outil (2) est constitué d'un corps principal (2**a**) allongé et creux à l'intérieur duquel sont agencés et guidés les premier et second moyens de connexion (3) et (5) et les moyens d'entrainement (7) de l'outil de coupe (6), ledit corps principal (2**a**) étant solidaire perpendiculairement à l'une de ses extrémité d'une première poignée fixe (2**b**) à l'intérieur de laquelle est logé les moyens de réglage (11), tandis qu'une seconde poignée mobile (2**c**) chargée élastiquement pivote autour de la poignée fixe (2**b**) afin de pouvoir agir sur les moyens d'entrainement (7) commandant les déplacements en translation longitudinale et en rotation de l'outil de coupe à lame (6).

10. Appareil de nucléotomie suivant les revendications 1 et 9, **caractérisé en ce que** les premiers moyens de connexion (3) sont constitués d'un manchon cylindrique de liaison (3**a**) fixé et guidé à l'intérieur du corps principal (2**a**), ledit manchon de liaison (3**a**) comportant un axe de liaison (3**b**) coopérant d'une part avec un axe de sortie (4**a**) du moteur électrique (4) et d'autre part avec les moyens d'entrainement (7) pour assurer l'entrainement en rotation de l'outil de coupe (6).

11. Appareil de nucléotomie suivant les revendications 1, 4 et 9, **caractérisé en ce que** les seconds moyens de connexion (5) sont constitués au niveau de l'extrémité libre du corps principal (2**a**) du porte outil 2 de deux encoches (5**a**, 5**b**) diamétralement opposées et destinées à recevoir respectivement par encliquetage des languettes de verrouillage (6**c**, 6**d**) réalisées dans la bague de connexion (6**e**) de l'outil de coupe (6).

12. Appareil de nucléotomie suivant la revendication 1, **caractérisé en ce que** les doigts de guidage (7**c**, 7**d**) débouchent à l'extérieur du corps principal (2**a**) pour être logés chacun à l'intérieur d'une rainure oblongue (2**h**, 2**i**) réalisée respectivement dans les branches (2**j** et 2**k**) de la fourche (2**l**) solidaire de la poigné mobile (2**c**) pour assurer les déplacements en translation de l'outil de coupe (6).

13. Appareil de nucléotomie suivant la revendication 1, **caractérisé en ce que** le manchon de liaison (7**a**) comporte dans le prolongement de son axe de transmission (7**b**) et à l'opposée des premiers moyens de connexion (3) un alésage de transmission (7**e**) permettant la réception de l'élément de liaison (6**b**) de l'outil de coupe (6) afin de transmettre à ce dernier les mouvements de rotation issu du moteur électrique (4).

14. Appareil de nucléotomie suivant la revendication 2, **caractérisé en ce que** les moyens de réglages (11) sont constitués d'une vis filetée (11**a**) guidée en rotation à l'intérieur de la poignée fixe (2**b**) du porte outil (2) et solidaire à l'une de ses extrémité d'une molette (11**b**) permettant de positionner un curseur (11**c**) au niveau de graduations (11**d**) gravées de part et d'autre d'une ouverture oblongue (2**d**) aménagées dans la partie supérieure de ladite poignée fixe (2**b**), ladite vis (11**a**) coopère avec un levier (2**e**) relié à un axe de rotation (2**f**) autour duquel pivote la poignée mobile (2**c**) de sorte que l'entrainement en rotation de la vis (11**a**) permet de positionner le levier (2**e**) dans différentes position angulaire afin de pouvoir agir sur la course de pivotement de la poigné mobile (2**c**) et par conséquent de limiter les déplacements en translation des moyens d'entrainement (7).

15. Appareil de nucléotomie suivant les revendications 3, **caractérisé en ce que** les moyens de rétraction (12) sont constitués d'une gâchette (12**a**) guidée dans ses pivotement sur le corps principal (2**a**) de porte outil (2) de manière à pouvoir déplacer un levier (12**b**) solidaire de ladite gâchette (12**a**) et dont l'extrémité libre (12**c**) est logée à l'intérieur d'un alésage (2**m**) ménagé dans ledit corps principal (2**a**).

16. Appareil de nucléotomie suivant la revendication 1, **caractérisé en ce que** les moyens d'injection et d'aspiration (8) sont constitués d'un circuit d'injection (80) dans lequel circule du sérum physiologique pénétrant à l'intérieur de l'espace nucléique Es en cours de formation et d'un circuit d'aspiration (81) permettant la récupération du sérum physiologique et des débris produits par le couteau bilame (6**a**) de l'outil de coupe (6).

17. Appareil de nucléotomie suivant la revendication 16, **caractérisé en ce que** le circuit d'injection (80) est raccordé à une pompe d'injection (82) alimenté en sérum physiologique tandis que le circuit d'aspiration (81) est connecté à une pompe d'aspiration (83) comportant un réservoir (84) pour la récupération du sérum physiologique et des débris.

18. Appareil de nucléotomie suivant la revendication 1, **caractérisé en ce que** le moteur électrique (4) à entraînement rotatif et les moyens d'injection et d'aspiration (8) sont relié à un boitier de commande (85) piloté par l'utilisateur au moyen de pédales de commande (86).

19. Appareil de nucléotomie suivant la revendication 1, **caractérisé en ce que** les moyens d'injection et d'aspiration (8) sont constitués d'un connecteur de liaison (87) venant se fixer d'une part sur l'extrémité d'un tube articulé (9) préalablement implanté dans le corps de la vertèbre Va correspondante et d'autre part sur le corps principal (2**a**) du porte outil (2).

20. Appareil de nucléotomie suivant la revendication 19, **caractérisé en ce que** le connecteur de liaison (87) comporte des moyens de fixation (88) qui sont constitués d'une part d'un taquet (88**a**) venant coopérer avec la tête de fixation (9**a**) du tube articulé (9) et d'autre d'une pince articulée (88**b**) venant s'encliqueter autour du corps principal (2**a**) du porte outil (2).

21. Appareil de nucléotomie suivant les revendications 19, **caractérisé en ce que** le connecteur de liaison (87) comporte des prises de raccordement (87**a** et 87**b**) pour la fixation des circuits d'injection (80) et d'aspiration (81).

## Patentansprüche

1. Nukleotomie-Vorrichtung, die ausgehend von einem zunächst intraossären Weg, der zuvor in den Körper eines Wirbels Va gebohrt wird, die Erstellung eines Nucleus-Raums Es in einer Bandscheibe Di ermöglicht, die einen Werkzeughalter (2) aufweist, der mit ersten Verbindungsmitteln (3), die das Einsetzen eines Elektromotors (4) mit Drehantrieb ermöglichen, mit zweiten Verbindungsmitteln (5) zum Einsetzen eines Schneidwerkzeugs mit Klinge (6) versehen ist, wobei Antriebsmittel (7) dem Schneidwerkzeug (6) Drehbewegungen und translatorische Längsbewegungen gewährleisten, **dadurch gekennzeichnet, dass** die Antriebsmittel (7) aus einer zylindrischen Verbindungshülse (7a) gebildet sind, die translatorisch im Inneren des Hauptkörpers (2a) geführt ist und einerseits in ihrem inneren Teil eine frei drehbare Übertragungswelle (7b), die mit der Verbindungswelle (3b) der ersten Verbindungsmittel (3) zusammenwirkt, um das Drehantreiben des Schneidwerkzeugs (6) zu gewährleisten, und andererseits an ihrem Außenumfang und einander gegenüberliegend Führungsstifte (7c, 7d) aufweist, die jeweils eine längliche Nut (2g) durchqueren, die in dem Hauptkörper (2a) des Werkzeughalters (2) ausgebildet ist, um mit den Zinken (2j und 2k) einer Gabel (21) zusammenzuwirken, die mit einem beweglichen Griff (2c) fest verbunden ist, um die translatorischen Bewegungen des Schneidwerkzeugs (6) und der Einspritz- und Ansaugmittel (8) zu gewährleisten, die ermöglichen, die Reste des Nucleus, die beim Schneiden bei ihrem Anordnen im Inneren der Bandscheibe entstanden sind, abzuführen.

2. Nukleotomie-Vorrichtung nach Anspruch 1, **dadurch gekennzeichnet, dass** sie Einstellmittel (11) aufweist, die den Lauf der translatorischen Bewegungen des Schneidwerkzeugs (6) im Inneren der Bandscheibe begrenzen.

3. Nukleotomie-Vorrichtung nach Anspruch 1, **dadurch gekennzeichnet, dass** sie Mittel zum Rückziehen (12) des Schneidwerkzeugs (6) vor seinem Einführen in die Bandscheibe aufweist.

4. Nukleotomie-Vorrichtung nach Anspruch 1, **dadurch gekennzeichnet, dass** das Schneidwerkzeug (6) aus einem flexiblen Metallstab (6f), der von einer Schutzhülle (6j) aus Kunststoff umhüllt ist, aus einer flexiblen Verbindung (6g), die ermöglicht, eines der Enden des Metallstabs (6f) mit einem Zwei-Klingen-Messer (6a), das aus einem superelastischen Material erstellt ist, zu verbinden, aus einem Verbindungselement (6b), das das andere Ende des Metallstabs (6f) mit den Antriebsmitteln (7) des Werkzeughalters (2) verbindet, und einem Verbindungsring (6e) gebildet ist, der mit den zweiten Verbindungsmitteln (5) zum Immobilisieren des Schneidwerkzeugs (6) auf dem Werkzeughalter (2) zusammenwirkt.

5. Nukleotomie-Vorrichtung nach Anspruch 4, **dadurch gekennzeichnet, dass** der Verbindungsring (6e) mit einem Spannfutter (6i) zusammenwirkt, das das Anordnen und das Halten der Schutzhülle (6j) um den flexiblen Metallstab (6f) herum ermöglicht.

6. Nukleotomie-Vorrichtung nach Anspruch 4, **dadurch gekennzeichnet, dass** die flexible Verbindung (6g) des Schneidwerkzeugs (6) aus einem Torsionsdraht (6k) gebildet ist, der einerseits an dem Zwei-Klingen-Messer (6a) und andererseits an dem Metallstab (6f) befestigt ist, wobei sich der Torsionsdraht (6k) verformen kann, um geringen Biegeradien zu folgen und dabei gleichzeitig dem Zwei-Klingen-Messer (6a) einen Drehantrieb mit hoher Drehzahl garantiert.

7. Nukleotomie-Vorrichtung nach Anspruch 6, **dadurch gekennzeichnet, dass** der Torsionsdraht (6k) mit dem Zwei-Klingen-Messer (6a) über eine mechanische Verbindung (6l) verbunden ist, die einerseits das Verschrauben des Zwei-Klingen-Messers (6a) und andererseits das Befestigen an dem Torsionsdraht (6k) durch Schweißen ermöglicht.

8. Nukleotomie-Vorrichtung nach Anspruch 7, **dadurch gekennzeichnet, dass** die mechanische Verbindung (6l) aus einer Hülse gebildet ist, die an einem ihrer Enden eine Gewindeinnenbohrung (6m) aufweist, die dazu bestimmt ist, mit einem Gewindeabschnitt (6n) des Zwei-Klingen-Messers (6a) zusammenzuwirken, während sich das andere Ende der Hülse durch eine Schulter (6p) mit geringem Durchmesser verlängert, die im Inneren des Torsionsdrahts (6k) aufgenommen wird, um an diesem Letzteren durch Schweißen befestigt zu werden.

9. Nukleotomie-Vorrichtung nach Anspruch 1, **dadurch gekennzeichnet, dass** der Werkzeughalter (2) aus einem länglichen und hohlen Hauptkörper (2a) gebildet ist, in dessen Innerem das erste und das zweite Verbindungsmittel (3) und (5) und die Antriebsmittel (7) des Werkzeughalters (2) angeordnet und geführt sind, wobei der Hauptkörper (2a) senkrecht zu einem seiner Enden mit einem ersten festen Griff (2b) fest verbunden ist, in dessen Inneren die Einstellmittel (11) untergebracht sind, während sich ein zweiter beweglicher Griff (2c), der federbelastet ist, um den festen Griff (2b) schwenkbewegt, um auf die Antriebsmittel (7) einzuwirken, die die translatorischen Längsbewegungen und die Drehbewegungen des Schneidwerkzeugs (6) steuern.

10. Nukleotomie-Vorrichtung nach Anspruch 1 und 9, **dadurch gekennzeichnet, dass** die ersten Verbindungsmittel (3) aus einer zylindrischen Verbindungshülse (3a) gebildet sind, die im Inneren des Hauptkörpers (2a) befestigt und geführt ist, wobei die Verbindungshülse (3a) eine Verbindungswelle (3b) aufweist, die einerseits mit einer Ausgangswelle (4a) des Elektromotors (4) und andererseits mit den Antriebsmitteln (7) zusammenwirkt, um den Drehantrieb des Schneidwerkzeugs (6) zu gewährleisten.

11. Nukleotomie-Vorrichtung nach Anspruch 1, 4 und 9, **dadurch gekennzeichnet, dass** die zweiten Verbindungsmittel (5) an dem freien Ende des Hauptkörpers (2a) des Werkzeughalters 2 aus zwei Einkerbungen (5a, 5b) bestehen, die diametral gegenüberliegend sind und dazu bestimmt sind, jeweils durch Einrasten Verriegelungszungen (6c, 6d) aufzunehmen, die in dem Verbindungsring (6e) des Schneidwerkzeugs (6) erstellt sind.

12. Nukleotomie-Vorrichtung nach Anspruch 1, **dadurch gekennzeichnet, dass** die Führungsstifte (7c, 7d) außerhalb des Hauptkörpers (2a) münden, um jeweils innerhalb einer länglichen Nut (2h, 2i) untergebracht zu werden, die jeweils in den Zinken (2j und 2k) der Gabel (21) erstellt ist, die mit dem beweglichen Griff (2c) fest verbunden ist, um die translatorischen Bewegungen des Schneidwerkzeugs (6) zu gewährleisten.

13. Nukleotomie-Vorrichtung nach Anspruch 1, **dadurch gekennzeichnet, dass** die Verbindungshülse (7a) in der Verlängerung ihrer Übertragungswelle (7b) und gegenüberliegend von den ersten Verbindungsmitteln (3) eine Übertragungsbohrung (7e) aufweist, die das Aufnehmen des Verbindungselements (6b) des Schneidwerkzeugs (6) ermöglicht, um auf dieses Letztere die Drehbewegungen zu übertragen, die aus dem Elektromotor (4) hervorgegangen sind.

14. Nukleotomie-Vorrichtung nach Anspruch 2, **dadurch gekennzeichnet, dass** die Einstellmittel (11) aus einer Gewindeschraube (11a) bestehen, die im Inneren des festen Griffs (2b) des Werkzeughalters (2) drehbar geführt ist und an einem ihrer Enden mit einem Rändelrad (11b) fest verbunden ist, das ermöglicht, einen Cursor (11c) an Graduierungen (11d) zu positionieren, die beiderseits einer länglichen Öffnung (2d) eingraviert sind, die in dem oberen Teil des festen Griffs (2b) ausgebildet sind, wobei die Schraube (11a) mit einem Hebel (2e) zusammenwirkt, der mit einer Drehwelle (2f) verbunden ist, um die sich der bewegliche Griff (2c) derart schwenkbewegt, dass das Indrehungversetzen der Schraube (11a) ermöglicht, den Hebel (2e) in verschiedenen Winkelpositionen zu positionieren, um auf den Schwenkweg des beweglichen Griffs (2c) einzuwirken und folglich die translatorischen Bewegungen der Antriebsmittel (7) zu begrenzen.

15. Nukleotomie-Vorrichtung nach Anspruch 3, **dadurch gekennzeichnet, dass** die Mittel zum Rückziehen (12) aus einem Auslöser (12a) gebildet sind, der in seinen Schwenkbewegungen auf dem Hauptkörper (2a) des Werkzeughalters (2) derart geführt ist, um einen Hebel (12b) zu bewegen, der mit dem Auslöser (12a) fest verbunden ist und dessen freies Ende (12c) im Inneren einer Bohrung (2m) untergebracht ist, die in dem Hauptkörper (2a) ausgebildet ist.

16. Nukleotomie-Vorrichtung nach Anspruch 1, **dadurch gekennzeichnet, dass** die Einspritz- und Ansaugmittel (8) aus einem Einspritzkreis (80), in dem physiologische Kochsalzlösung fließt, die ins Innere des sich in Bildung befindlichen Nucleus-Raums Es eindringt, und einem Ansaugkreis (81) gebildet sind, der das Rückgewinnen der physiologischen Kochsalzlösung und der Reste, die durch das Zwei-Klingen-Messer (6a) des Schneidwerkzeugs (6) erzeugt werden, ermöglicht.

17. Nukleotomie-Vorrichtung nach Anspruch 16, **dadurch gekennzeichnet, dass** der Einspritzkreis (80) an eine Einspritzpumpe (82) angeschlossen ist, die mit physiologischer Kochsalzlösung gespeist wird, während der Ansaugkreis (81) mit einer Ansaugpumpe (83) verbunden ist, die einen Behälter (84) für das Rückgewinnen der physiologischen Kochsalzlösung und der Reste aufweist.

18. Nukleotomie-Vorrichtung nach Anspruch 1, **dadurch gekennzeichnet, dass** der Elektromotor (4) mit Drehantrieb und die Einspritz- und Ansaugmittel (8) mit einem Steuerkasten (85) verbunden sind, der von dem Benutzer mit Hilfe von Steuerungspedalen (86) gesteuert wird.

19. Nukleotomie-Vorrichtung nach Anspruch 1, **dadurch gekennzeichnet, dass** die Einspritz- und Ansaugmittel (8) aus einem Verbindungsstecker (87) gebildet sind, der einerseits an dem Ende eines Gelenkrohrs (9), das zuvor in den Körper des entsprechenden Wirbels Va implantiert worden ist, und andererseits an dem Hauptkörper (2a) des Werkzeughalters (2) befestigt wird.

20. Nukleotomie-Vorrichtung nach Anspruch 19, **dadurch gekennzeichnet, dass** der Verbindungsstecker (87) Befestigungsmittel (88) aufweist, die einerseits aus einer Nase (88a), die mit dem Befestigungskopf (9a) des Gelenkrohrs (9) zusammenwirkt, und andererseits aus einer Gelenkzange (88b) gebildet sind, die um den Hauptkörper (2a) des Werkzeughalters (2) einrastet.

21. Nukleotomie-Vorrichtung nach Anspruch 19, **dadurch gekennzeichnet, dass** der Verbindungsstecker (87) Anschlussdosen (87a und 87b) zum Befestigen des Einspritz- (80) und des Ansaugkreises (81) aufweist.

## Claims

1. Nucleotomy device making it possible, from a trans-osseous approach previously pierced in the body of a vertebra Va, to produce a nucleic space Es in an intervertebral disc Di, which device comprises a tool holder (2) provided with first connection means (3) ensuring the placement of an electric rotary driving motor (4), second connection means (5) for the placement of a blade cutting tool (6), drive means (7) imparting rotational and longitudinal translational movements to the cutting tool (6), **characterized in that** said drive means (7) are made up of a cylindrical linking sleeve (7a) guided in translation inside the main body (2a) and comprising, on the one hand, in its inner part, a freely rotating transmission shaft (7b) which cooperates with the linking shaft (3b) of the first connection means (3) to ensure rotational driving of the cutting tool (6), and, on the other hand, on its outer periphery and opposite one another, guide fingers (7c, 7d), respectively passing through an oblong groove (2g) formed in the main body (2a) of the tool holder (2) to cooperate with branches (2j and 2k) of a fork (21) secured to a movable handle (2c) to ensure the translational movements of the cutting tool (6) and of the injection and suction means (8) making it possible to evacuate the nucleus debris resulting from the cut during the performing of the latter inside the intervertebral disc.

2. Nucleotomy device according to claim 1, **characterized in that** it comprises adjustment means (11) limiting the translational travel of movement of the cutting tool (6) inside the intervertebral disc.

3. Nucleotomy device according to claim 1, **characterized in that** it comprises retraction means (12) for retracting the cutting tool before it is inserted into the intervertebral disc.

4. Nucleotomy device according to claim 1, **characterized in that** the cutting tool (6) is made up of a flexible metal rod (6f) enclosed in a protective sheath (6j) of plastic material, of a flexible link (6g) making it possible to link one end of the metal rod (6f) to a two-blade knife (6a) made from a super-elastic material, of a linking element (6b) linking the other end of the metal rod (6f) to the drive means (7) of the tool holder (2) and a connection ring (6e) cooperating with the second connection means (5) to immobilize said cutting tool (6) on said tool holder (2).

5. Nucleotomy device according to claim 4, **characterized in that** the connection ring (6e) cooperates with a clamping mandrel (6i) making it possible to place and retain the protective sheath (6j) around the flexible metal rod (6f).

6. Nucleotomy device according to claim 4, **characterized in that** the flexible link (6g) of the cutting tool (6) is made up of a torsion cable (6k) fixed on the one hand to the two-blade knife (6a) and on the other hand to the metal rod (6f), said torsion cable (6k) being capable to deform to follow small radii of curvature while imparting a high-speed a rotational driving to said two-blade knife (6a).

7. Nucleotomy device according to claim 6, **characterized in that** the torsion cable (6k) is connected to the two-blade knife (6a) by means of a mechanical link (6l) making it possible on the one hand to screw said two-blade knife (6a) and on the other hand to fasten it to said torsion cable (6k) by welding.

8. Nucleotomy device according to claim 7, **characterized in that** the mechanical link (61) is made up of a sleeve comprising at one of its ends a threaded inner bore (6m) designed to cooperate with a threaded part (6n) of the two-blade knife (6a), while the other end of the sleeve is extended by a shoulder (6p) of small diameter which is housed inside the torsion cable (6k) to be fastened to the latter by welding.

9. Nucleotomy device according to claim 1, **characterized in that** the tool holder (2) is made up of an elongated and hollow main body (2a) inside which the first and second connection means (3) and (5) and the drive means (7) for driving the cutting tool (6) are arranged and guided, said main body (2a) being secured perpendicular to one of its ends to a first stationary handle (2b) inside which the adjustment means (11) are housed, while a second elastically charged movable handle (2c) pivots around the stationary handle (2b) so as to be capable to act on the drive means (7) controlling the longitudinal translational and rotational movements of the blade cutting tool (6).

10. Nucleotomy device according to claims 1 and 9, **characterized in that** the first connection means (3) are made up of a cylindrical linking sleeve (3a) fastened and guided inside the main body (2a), said linking sleeve (3a) comprising a linking shaft (3b) cooperating on the one hand with an output shaft (4a) of the electric motor (4) and on the other hand with the drive means (7) to ensure the rotary drive of the cutting tool (6).

11. Nucleotomy device according to claims 1, 4 and 9, **characterized in that** the second connection means (5) are formed at the free end of the main body (2a) of the tool holder (2) of two diametrically opposite notches (5a, 5b) respectively designed to receive, by snap-fastening, locking tabs (6c, 6d) formed in the connection ring (6e) of the cutting tool (6).

12. Nucleotomy device according to claim 1, **characterized in that** the guide fingers (7c, 7d) open out to the outside of the main body (2a) to be each housed inside an oblong groove (2h, 2i) respectively formed in the branches (2j and 2k) of the fork (21) secured to the movable handle (2c) to ensure the translational movements of the cutting tool (6).

13. Nucleotomy device according to claim 1, **characterized in that** the linking sleeve (7a) comprises, in the extension of its transmission shaft (7b) and opposite the first connection means (3), a transmission bore (7e) making it possible to receive the linking element (6b) of the cutting tool (6) so as to impart the rotational movements coming from the electric motor (4) to the latter.

14. Nucleotomy device according to claim 2, **characterized in that** the adjustment means (11) are made up of a threaded screw (11a) guided in rotation inside the stationary handle (2b) of the tool holder (2) and secured at one of its ends to a knob (11b) for positioning a slider (11c) at the level of graduations (11d) engraved on either side of an oblong opening (2d) formed in the upper part of said stationary handle (2b), said screw (11a) cooperates with a lever (2e) connected to a rotation pin (2f) around which the movable handle (2c) pivots, such that the rotational driving of the screw (11a) makes it possible to position the lever (2e) in different angular positions in order to be capable to act on the pivoting stroke of the movable handle (2c) and consequently to limit the translational movements of the drive means (7).

15. Nucleotomy device according to claims 3, **characterized in that** the retraction means (12) are made up of a trigger (12a) guided in its pivoting on the main body (2a) of the tool holder (2) so as to be capable to move a lever (12b) secured to said trigger (12a) and the free end (12c) of which is housed inside a bore (2m) formed in said main body (2a).

16. Nucleotomy device according to claim 1, **characterized in that** the injection and suction means (8) are made up of an injection circuit (80) in which physiological serum circulates penetrating inside the nucleic space Es being formed and a suction circuit (81) making it possible to recover the physiological serum and the debris produced by the two-blade knife (6a) of the cutting tool (6).

17. Nucleotomy device according to claim 16, **characterized in that** the injection circuit (80) is connected to an injection pump (82) supplied with physiological serum while the suction circuit (81) is connected to a suction pump (83) comprising a reservoir (84) for recovering physiological serum and debris.

18. Nucleotomy device according to claim 1, **characterized in that** the electric rotational drive motor (4) and the injection and suction means (8) are connected to a control unit (85) controlled by the user by means of control pedals (86).

19. Nucleotomy device according to claim 1, **characterized in that** the injection and suction means (8) are made up of a linking connector (87) which is fastened on the one hand on the end of a hinged tube (9) previously implanted in the body of the corresponding vertebra Va and on the other hand on the main body (2a) of the tool holder (2).

20. Nucleotomy device according to claim 19, **characterized in that** the linking connector (87) comprises fastening means (88) that are made up, on the one hand, of a cleat (88a) that cooperates with the fastening head (9a) of the hinged tube (9) and, on the other hand, a hinged clamp (88b) snapping around the main body (2a) of the tool holder (2).

21. Nucleotomy device according to claims 19, **characterized in that** the linking connector (87) comprises connection sockets (87a and 87b) for fixing the injection (80) and suction (81) circuits.
